(19) Europäisches Patentamt / European Patent Office / Office européen des brevets

(11) **EP 4 355 942 B1**

(12) **EUROPÄISCHE PATENTSCHRIFT**

(45) Veröffentlichungstag und Bekanntmachung des Hinweises auf die Patenterteilung:
**19.02.2025 Patentblatt 2025/08**

(21) Anmeldenummer: **22761974.9**

(22) Anmeldetag: **02.08.2022**

(51) Internationale Patentklassifikation (IPC):
*D04H 1/541* (2012.01)    *D04H 1/407* (2012.01)
*D04H 1/413* (2012.01)    *D04H 1/56* (2006.01)
*D04H 3/147* (2012.01)    *A61F 13/537* (2006.01)

(52) Gemeinsame Patentklassifikation (CPC):
**D04H 1/5418; A61F 13/537; D04H 1/407; D04H 1/413; D04H 1/4291; D04H 1/4358; D04H 1/56; D04H 3/147**

(86) Internationale Anmeldenummer:
**PCT/EP2022/071755**

(87) Internationale Veröffentlichungsnummer:
**WO 2023/012191 (09.02.2023 Gazette 2023/06)**

(54) **VERFAHREN ZUR HERSTELLUNG EINES POLYMER-VLIESSTOFFS**

METHOD FOR MANUFACTURING A POLYMER NONWOVEN FABRIC

PROCÉDÉ DE FABRICATION D'UN NON-TISSÉ POLYMÈRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.08.2021 DE 102021208606**

(43) Veröffentlichungstag der Anmeldung:
**24.04.2024 Patentblatt 2024/17**

(73) Patentinhaber: **Friedrich-Alexander-Universität Erlangen-Nürnberg, Körperschaft des öffentlichen Rechts**
**91054 Erlangen (DE)**

(72) Erfinder:
• **DAENICKE, Jonas**
**90409 Nürnberg (DE)**
• **SCHUBERT, Dirk Wolfram**
**91330 Eggolsheim (DE)**

(74) Vertreter: **FDST Patentanwälte**
**Nordostpark 16**
**90411 Nürnberg (DE)**

(56) Entgegenhaltungen:
**EP-A1- 2 292 822      EP-A1- 2 463 428**
**WO-A1-2017/095399   WO-A1-2019/092166**
**CN-A- 112 281 313**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft ein Verfahren zur Herstellung eines Polymer-Vliesstoffs. Des Weiteren betrifft die Erfindung eine Verwendung eines Vlies-Halbzeugs, das dem Polymer-Vliesstoff zugrunde liegt, sowie eine Verwendung des Polymer-Vliesstoffs.

Polymer-Vlies(stoff)e kommen heutzutage in vielen unterschiedlichen Anwendungen zum Einsatz. Sie werden dabei auch häufig als "non-wovens" bezeichnet, um sie von ihrer Struktur her von anderen "Textilen", insbesondere Geweben und Gewirken, abzugrenzen. Grundsätzlich können solche Polymer-Vliesstoffe im Rahmen von Faserverstärkungen zum Einsatz kommen, bspw. als oberflächliche Laminat-Schicht, um eine möglichst glatte Oberfläche mit geringen Abzeichnungen von Faser-Ondulationen auszubilden, oder als näherungsweise isotrope Verstärkungslage. Einsatz finden sie auch als Filtermaterial, bspw. bei Atemschutzmasken aber auch bspw. bei Staubsauger-Beuteln oder austauschbaren Filtern.

Interessant sind aber auch Anwendungen mit dem Zweck der Flüssigkeitsaufnahme und/oder Weitergabe. Hier kommen sie bspw. im Bereich von (Baby-) Windeln oder dergleichen als sogenannte Aquisition Distribution Layer ("ADL") zum Einsatz. Deren Aufgabe ist es, Flüssigkeit aufgrund einer guten Saugfähigkeit aufzunehmen und flächig verteilt an ein dahinterliegendes Speichermedium, meist ein Gel, abzugeben.

Insbesondere für letzteren Zweck werden vergleichsweise voluminöse Vliesstrukturen angestrebt. Aus dem Stand der Technik sind bspw. aus US 2019/ 0 388 263 A1 oder auch US 2019/ 0 183 690 A1 Schichtaufbauten bekannt, bei denen ein solches Polymer-Vlies an mehreren Stellen auf ein elastisches Trägermaterial aufgeklebt wird, während sich dieses in einem gedehnten Zustand befindet. Bei anschließender Entspannung zieht sich das Trägermaterial zusammen, wodurch das Polymer-Vlies durch Wellen- oder Faltenbildung "aufplustert".

Außerdem werden ADLs häufig auch aus kardierten Fasern hergestellt, was vergleichsweise aufwendig ist.

Der Erfindung liegt die Aufgabe zugrunde, die Herstellung eines Polymer-Vliesstoffs zu verbessern.

Diese Aufgabe wird erfindungsgemäß gelöst durch ein Verfahren mit den Merkmalen des Anspruch 1. Des Weiteren wird diese Aufgabe erfindungsgemäß gelöst durch eine Verwendung eines Vlies-Halbzeugs gemäß Anspruch 11 sowie durch eine Verwendung eines Polymer-Vliesstoffs gemäß Anspruch 12. Weitere vorteilhafte und für sich erfinderische Ausführungsformen und Weiterentwicklungen der Erfindung sind in den Unteransprüchen und der nachfolgenden Beschreibung dargelegt.

Das erfindungsgemäße Verfahren dient zur Herstellung eines Polymer-Vliesstoffs. Verfahrensgemäß wird dabei zunächst ein Polymerblend aus einem thermoplastischen Elastomer (im Folgenden als "TPE" bezeichnet) und einem weiteren thermoplastischen Material bereitgestellt. Aus diesem Polymerblend werden Fasern erzeugt ("gesponnen") und daraus, insbesondere dabei, ein Vlies-Halbzeug gebildet. Der Polymer-Vliesstoff wird erzeugt, indem anschließend das Vlies-Halbzeug mechanisch verstreckt wird. Durch darauffolgende Entspannung (d. h. Wegnahme der mechanischen Verstreckung) wird dabei eine Volumenzunahme erzeugt (hervorgerufen).

Anders ausgedrückt wird zur Herstellung des Polymer-Vliesstoffs ein aus dem Polymerblend gebildetes (insbesondere herkömmliches Spinn-) Vlies (-Halbzeug) gedehnt und anschließend wieder entspannt. Durch dieses Dehnen und anschließende Entspannen erfährt das Vlies die vorgenannte Volumenzunahme. Der Effekt der Volumenzunahme beruht hierbei insbesondere auf einer plastischen und somit nicht reversiblen Dehnung der Anteile des weiteren thermoplastischen Materials innerhalb der durch den Polymerblend gebildeten Fasern, wohingegen die Anteile des TPE elastisch rückstellen. Dabei kommt es zum Biegen der plastisch deformierten Anteile und damit der gesamten Faser, was zu einer Volumenzunahme im Vlies führt.

Bevorzugt kommt dabei nur eine Faserart, nämlich die aus dem Polymerblend gebildete, zum Einsatz. Bei dem Vlies-Halbzeug handelt es sich also nicht um ein Laminat, ein Multifaser-Vlies oder dergleichen. Besonders bevorzugt ist diese Faserart, also die Fasern für das Vlies-Halbzeug nur aus dem Polymerblend gebildet.

Dieses Vorgehen hat dabei den Vorteil, dass das Vlies-Halbzeug eine für (Spinn-) Vliese - insbesondere direkt nach der Fertigung des Vlieses - herkömmliche Dicke aufweist und somit besonders gute Lagerungs- und Transporteigenschaften. Im Gegensatz zu herkömmlichen voluminösen Polymer-Vliesstoffen, die bereits bei deren Fertigung ihr Ziel-Volumen - insbesondere deren Ziel-Dicke - erreichen, kann hier auf einfache und vorteilhafte Weise die Ausbildung des Ziel-Volumens an den Ort der Weiterverarbeitung verlegt werden, da lediglich eine Dehnung des Vlies-Halbzeugs erforderlich ist, im Gegensatz zu vergleichsweise aufwendigen Laminierprozessen.

In einer bevorzugten Verfahrensvariante wird als TPE ein thermoplastisches Polyurethan (im Folgenden als "TPU" bezeichnet) herangezogen.

In einer zweckmäßigen Verfahrensvariante wird als weiteres thermoplastisches Material ein solches herangezogen, das zu den Komponenten des (meist als (Block-)Copolymer ausgebildeten) TPE eine Löslichkeitsparameterdifferenz von größer 1, vorzugsweise von größer $2 \left( \frac{J}{cm^3} \right)^{1/2}$ aufweist.

In einer weiteren zweckmäßigen Verfahrensvariante wird als weiteres thermoplastisches Material ein zu dem TPE, insbesondere TPU, inkompatibler Thermoplast herangezogen. Als "inkompatibel" wird hier und im Folgenden insbesondere verstanden, dass das weitere thermoplastische Material keine gemeinsame Phase mit dem TPE, insbesondere TPU, in dem Polymerblend ausbildet, sondern als eigenständige Phase neben der TPE, insbesondere TPU, -Phase vorliegt. Dies wird insbesondere aufgrund der vorstehend beschriebenen Löslichkeitsparameterdifferenz erreicht oder ermöglicht. Insbesondere wird ein Polypropylen (PP) oder ein Polyethylen (PE) herangezogen.

Vorzugsweise wird dabei auch ein gegenüber dem TPE, insbesondere TPU, steiferes, insbesondere vergleichsweise unelastisches, Material (insbesondere also ein herkömmlicher Thermoplast - quasi ein "Nicht-TPE-Thermoplast") herangezogen.

In einer zweckmäßigen Verfahrensvariante wird das weitere thermoplastische Material mit einem Anteil von 1,5 bis 30 Gew.-%, insbesondere von 5 bis 20 Gew.-%, dem TPE bzw. TPU beigemengt.

Außerdem werden die aus dem Polymerblend gebildeten Fasern in einer weiteren zweckmäßigen Verfahrensvariante bei deren Erzeugung, zumindest bei der Bildung des Vlies-Halbzeugs, verstreckt, insbesondere so dass im Spinnprozess Fibrillen der ausgeschiedenen Phasen des weiteren thermoplastischen Materials gebildet werden. Plakativ ausgedrückt bilden die ausgeschiedenen Phasen des weiteren thermoplatischen Materials "Kleinst-Fasern" innerhalb der aus dem Polymerblend gebildeten Fasern, also eine Art im TPE eingebettete "Sub-Fasern".

Insbesondere werden die Fasern mittels eines sogenannten "melt-blown" Prozesses hergestellt und dabei auch das Vlies-Halbzeug beim Ablegen der hergestellten Fasern ausgebildet. Dabei hat insbesondere das TPU den Vorteil, dass die Fasern beim Ablegen selbstbindend sind und somit der Zusammenhalt des Vlies-Halbzeugs ermöglicht werden kann. Dies liegt insbesondere daran, dass die Fasern im melt-blown Prozess aufgrund der Heißgas-Zufuhr beim Ablegen noch klebrig sind. Typische Nachverfestigungsverfahren wie air through drying, Kalandrierung, needle punch etc. sind hierdurch vorteilhafterweise nicht erforderlich.

Alternativ ist eine Ausbildung der Fasern und des Vlies-Halbzeugs mittels anderer Spinnverfahren, insbesondere mittels Schmelzspinnen, ebenfalls möglich. Auch hier erfolgt eine Verstreckung der Fasern bei deren Erzeugung, also im Rahmen des Spinnverfahrens. In diesem Fall erfolgt insbesondere eine konventionelle Nachverfestigung der (insbesondere wirr aufeinander) abgelegten Fasern zum Vlies-Halbzeug. Diese Nachverfestigung - bspw. lokales (punktuelles) Kalandrieren, air through drying oder needle punch - erfolgt dabei vor der nachfolgenden (oder späteren) Verstreckung des Vlies-Halbzeugs, die zur Volumenerhöhung dient.

In einer weiteren zweckmäßigen Verfahrensvariante wird durch die mechanische Streckung des Vlies-Halbzeugs auch eine Porengröße erhöht. Mit anderen Worten werden die Poren des Vlies-Halbzeugs (insbesondere ebenfalls oder zusätzlich zum Volumen) vergrößert. Dies ist wiederum vorteilhaft für den Einsatz des Polymer-Vliesstoffs als ADL.

In einer besonders zweckmäßigen Verfahrensvariante wird die Dicke des Vlies-Halbzeugs aufgrund der mechanischen Streckung und somit auch der Volumenerhöhung um das 2 bis 10-fache, insbesondere bis um das 8-Fache insbesondere im Vergleich zur Ausgangsdicke des Vlies-Halbzeugs erhöht. Dadurch kann bspw. der für Lagerung und/oder Transport erforderliche Raum verringert, oder umgekehrt eine größere Halbzeugmenge eingelagert bzw. transportiert werden.

Bei der mechanischen Streckung wird in einer zweckmäßigen Verfahrensvariante, insbesondere zur Einstellung der vorstehend beschriebenen Volumen- und Eigenschaftsänderungen des Vlies-Halbzeugs, das Vlies-Halbzeug um mindestens 20, 25 oder 50 Prozent und bis zu 80 Prozent, insbesondere bis 75 Prozent (insbesondere gegenüber seiner Ausgangslänge) gestreckt.

Das weitere thermoplastische Material wird dabei vorzugsweise derart gewählt, dass dieses bei diesen Deformationsgraden (also den vorstehend genannten prozentualen Verstreckungen) plastisch deformiert bleibt, wohingegen das TPE elastisch rückstellt.

Versuche wurden unter anderem durchgeführt mit einem TPU der Marke Elasto-Ilan (eingetragene Marke der BASF Polyurethanes GmbH), Type 1180A und als weiterem (thermoplastischen) Kunststoff ein Polypropylen (PP) der Type HL504FB (Borealis AG) mit einem Anteil von 5 Gew.-% oder ein PP der Marke Moplen (eingetragene Marke der LyondellBasell Industries Holdings B.V.), Type HP561r mit einem Anteil von 20 Gew.-%. Dabei konnte nach der mechanischen Verstreckung - hier mit Verstreckungen von etwa 25 und 38 Prozent - eine etwa fünf- bis sogar etwa achtfache Vliesstoffdicke festgestellt werden.

Beispielsweise wurden bei einer Ausgangsdicke des Vlies-Halbzeugs von 0,85 Millimeter nach der Verstreckung eine Dicke von etwa 6,58 Millimeter für den Polymer-Vliesstoff ermittelt.

Die Erfindung sieht auch die Verwendung des vorstehend beschriebenen Vlies-Halbzeugs zur Herstellung eines Polymer-Vliesstoffs, insbesondere für den Einsatz als ADL, vor. Mithin wird das Vlies-Halbzeug wie vorstehend beschrieben aus Fasern gebildet, die aus dem Polymerblend erzeugt sind, das aus TPE, insbesondere TPU, und einem weiteren thermoplastischen Material gebildet ist. Erfindungsgemäß wird das Vlies-Halbzeug mechanisch gestreckt und anschließend entspannt, wodurch die Volumenzunahme des Vlies-Halbzeugs hervorgerufen und der Polymer-Vliesstoff, der dann vorzugsweise als ADL zum Einsatz kommt, erzeugt wird.

Die Erfindung sieht - zusätzlich oder alternativ zur Verwendung des Polymer-Vliesstoffs als ADL - auch die Verwendung des gemäß vorstehend beschriebenem Verfahren hergestellten Polymer-Vliesstoffs als Träger von Katalysator-, Aktivkohle-, Superabsorberpartikeln und/oder dergleichen vor.

Die erfindungsgemäßen Verwendungen sowie das vorstehend beschriebene erfindungsgemäße Verfahren teilen alle vorstehend beschriebenen Verfahrensschritte und somit auch die sich daraus ergebenden körperlichen Merkmale sowie Vorteile.

Die Konjunktion "und/oder" ist hier und im Folgenden insbesondere derart zu verstehen, dass die mittels dieser Konjunktion verknüpften Merkmale sowohl gemeinsam als auch als Alternativen zueinander ausgebildet sein können.

Nachfolgend wird ein Ausführungsbeispiel der Erfindung anhand einer Zeichnung näher erläutert. Darin zeigen:

Fig. 1    in einer schematischen Darstellung einzelne Verfahrensschritte des Verfahrens, und

Fig. 2    in einem schematischen Ablaufdiagramm ein Verfahren zur Herstellung eines Polymer-Vliesstoffs.

Einander entsprechende Teile sind in allen Figuren stets mit gleichen Bezugszeichen versehen.

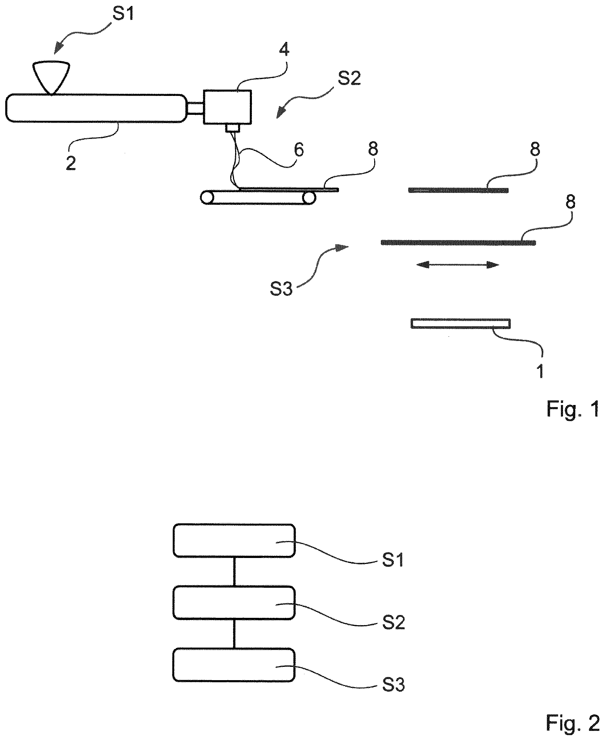

Anhand von Fig. 1 und 2 wird im Folgenden ein Verfahren zur Herstellung eines Polymer-Vliesstoffs, kurz Vliesstoff 1, dargestellt. Dazu wird in einem ersten Verfahrensschritt S1 ein Polymerblend aus einem thermoplastischen Elastomer, konkret einem thermoplastischen Polyurethan (TPU) und einem Polypropylen (PP) bereitgestellt. Im vorliegenden Ausführungsbeispiel wird PP mit einem Gewichtsanteil von 5 % dem TPU beigemengt. Mittels eines Extruders 2 wird das Polymerblend einer Spinnvorrichtung 4 zugeführt. Optional erfolgt das Blenden (d. h. das Erstellen des Polymerblends) auch erst in diesem Extruder 2.

Mittels der Spinnvorrichtung 4 werden aus dem Polymerblend in einem zweiten Verfahrensschritt S2 mittels der Spinnvorrichtung 4 Fasern 6 gesponnen, konkret mittels eines "melt-blown" Prozesses. Beim Ablegen der Fasern 6 wird direkt ein Vlies-Halbzeug (hier kurz: "Halbzeug 8") erzeugt. Konkret handelt es sich bei dem Halbzeug 8 bereits um ein Vlies im eigentlichen Sinnen, d. h. um wirr abgelegte und miteinander verbundene Fasern 6. Der Zusammenhalt der Fasern 6 in dem Halbzeug 8 wird dabei aufgrund des melt-blown Prozesses durch die noch "heißen" Fasern 6 und die damit noch klebrigen TPU-Anteile ermöglicht.

Das fertige Halbzeug 8 kann nun (zwischen-) gelagert oder zu einem Ort für die Weiterverarbeitung transportiert werden.

In einem dritten Verfahrensschritt S3, dieser kann und sollte vorzugsweise am Ort der Weiterverarbeitung erfolgen, wird das Halbzeug 8 mechanisch gestreckt. Das Halbzeug 8 wird dabei um etwa 40 % gestreckt. Nach der Streckung wird das Halbzeug 8 wieder entlastet. Bei der Entlastung stellt sich eine Volumenzunahme des Halbzeugs 8 ein, die sich in einer Vervielfachung, bspw. um das 5-Fache, der Dicke niederschlägt. Das so behandelte Halbzeug 8 bildet das Zielprodukt, den Vliesstoff 1. Dieser kann bspw. als sogenannter Aquisition Distribution Layer bspw. in Windeln oder dergleichen zum Einsatz kommen. Des Weiteren kann der Vliesstoff 1 aber auch als Träger von Katalysator-, Aktivkohle- und/oder Superabsorberpartikeln eingesetzt werden.

Der Gegenstand der Erfindung ist nicht auf das vorstehend beschriebene Ausführungsbeispiel beschränkt. Vielmehr können weitere Ausführungsformen der Erfindung von dem Fachmann aus der vorstehenden Beschreibung abgeleitet werden.

Bezugszeichenliste

1    Vliesstoff
2    Extruder
4    Spinnvorrichtung
6    Faser
8    Halbzeug

S1    Verfahrensschritt
S2    Verfahrensschritt
S3    Verfahrensschritt

**Patentansprüche**

1.  Verfahren zur Herstellung eines Polymer-Vliesstoffs (1), wobei verfahrensgemäß

    - ein Polymerblend aus einem thermoplastischen Elastomer und einem weiteren thermoplastischen Material bereitgestellt wird,
    - aus dem Polymerblend Fasern (6) erzeugt und daraus ein Vlies-Halbzeug (8) gebildet wird, und
    - der Polymer-Vliesstoff (1) erzeugt wird, indem durch mechanische Streckung des Vlies-Halbzeugs (8) und anschließende Entspannung eine Volumenzunahme erzeugt wird.

2.  Verfahren nach Anspruch 1,
    wobei als thermoplastisches Elastomer ein thermoplastisches Polyurethan herangezogen wird.

3.  Verfahren nach Anspruch 1 oder 2,
    wobei als weiteres thermoplastisches Material ein solches herangezogen wird, das zu Komponenten des thermoplastischen Elastomers eine Löslichkeitsparameterdifferenz von größer 1, vorzugsweise von größer $2\left(\frac{J}{cm^3}\right)^{1/2}$ aufweist.

4.  Verfahren nach einem der Ansprüche 1 bis 3,
    wobei als weiteres thermoplastisches Material ein zu dem thermoplastischen Elastomer inkompatibler Thermoplast, bspw. ein Polypropylen oder ein Polyethylen, herangezogen wird.

**5.** Verfahren nach einem der Ansprüche 1 bis 4, wobei das weitere thermoplastische Material mit einem Anteil von 1,5 bis 30 Gew.-%, insbesondere von 5 bis 20 Gew.-%, dem thermoplastischen Elastomer beigemengt wird.

**6.** Verfahren nach einem der Ansprüche 1 bis 5, wobei die Fasern (6) bei der Bildung des Vlies-Halbzeugs (8) verstreckt werden.

**7.** Verfahren nach einem der Ansprüche 1 bis 6, wobei das Vlies-Halbzeug (8) in einem melt-blown-Prozess hergestellt wird.

**8.** Verfahren nach einem der Ansprüche 1 bis 7, wobei durch die mechanische Streckung eine Porengröße erhöht wird.

**9.** Verfahren nach einem der Ansprüche 1 bis 8, wobei die Dicke des Vlies-Halbzeugs (8) aufgrund der mechanischen Streckung um das 2 bis 10-fache, insbesondere bis um das 8-Fache erhöht wird.

**10.** Verfahren nach einem der Ansprüche 1 bis 9, wobei das Vlies-Halbzeug (8) um 20 bis zu 80 Prozent, insbesondere 25 bis 75 Prozent, gestreckt wird.

**11.** Verwendung eines Vlies-Halbzeugs (8), das aus Fasern (6) gebildet ist, die aus einem Polymerblend erzeugt sind, das aus einem thermoplastischen Elastomer und einem weiteren thermoplastischen Material gebildet ist, zur Herstellung eines Polymer-Vliesstoffs (1), insbesondere eines aus diesem gebildeten Aquisition Disitribution Layer, wobei durch mechanische Streckung des Vlies-Halbzeugs (8) und anschließende Entspannung eine Volumenzunahme () des Vlies-Halbzeugs (8) hervorgerufen und der Polymer-Vliesstoff (1) erzeugt wird.

**12.** Verwendung eines Polymer-Vliesstoffs (1), der nach einem Verfahren gemäß einem der Ansprüche 1 bis 10 hergestellt ist, als Träger von Katalysator-, Aktivkohle- und/oder Superabsorberpartikeln.

**Claims**

**1.** Process for producing a polymer nonwoven (1), in which process

- a polymer blend of a thermoplastic polymer and a further thermoplastic material is provided,
- the polymer blend is used to produce fibres (6) and these are used to form a semifinished nonwoven product (8), and

- the polymer nonwoven (1) is produced by mechanical stretching of the semifinished nonwoven product (8) and subsequent relaxation in order to achieve an increase in volume.

**2.** Process according to Claim 1, wherein the thermoplastic elastomer employed is a thermoplastic polyurethane.

**3.** Process according to Claim 1 or 2, wherein the further thermoplastic material employed is one having a difference in solubility parameter with respect to components of the thermoplastic elastomer of greater than 1, preferably of greater than

$$2\left(\frac{J}{cm^3}\right)^{1/2}.$$

**4.** Process according to any of Claims 1 to 3, wherein the further thermoplastic material employed is a thermoplastic which is incompatible with the thermoplastic elastomer, for example a polypropylene or a polyethylene.

**5.** Process according to any of Claims 1 to 4, wherein the further thermoplastic material is added to the thermoplastic elastomer with a proportion of 1.5% to 30% by weight, especially 5% to 20% by weight.

**6.** Process according to any of Claims 1 to 5, wherein the fibres (6) are stretched in the forming of the semifinished nonwoven product (8).

**7.** Process according to any of Claims 1 to 6, wherein the semifinished nonwoven product (8) is produced in a meltblown process.

**8.** Process according to any of Claims 1 to 7, wherein the mechanical stretching increases a pore size.

**9.** Process according to any of Claims 1 to 8, wherein the thickness of the semifinished nonwoven product (8) is increased owing to the mechanical stretching by 2 to 10 times, especially by up to 8 times.

**10.** Process according to any of Claims 1 to 9, wherein the semifinished nonwoven product (8) is stretched by 20 up to 80 per cent, especially 25 to 75 per cent.

**11.** Use of a semifinished nonwoven product (8) formed from fibres (6) produced from a polymer blend which is formed from a thermoplastic elastomer and a further thermoplastic material in order to produce a polymer nonwoven (1), especially an acquisition/distribution layer formed therefrom, wherein mechanical stretching of the semifinished nonwoven product (8) and subsequent relaxation cause an increase in volume of the semifinished nonwoven product (8) and produce the polymer nonwoven (1).

12. Use of a polymer nonwoven (1) produced by a process according to any of Claims 1 to 10 as support for catalyst particles, activated carbon particles and/or superabsorbent particles.

**Revendications**

1. Procédé de fabrication d'un non-tissé polymère (1), dans lequel, selon le procédé,

   - on prépare un mélange de polymères à partir d'un élastomère thermoplastique et d'un autre matériau thermoplastique,
   - on produit des fibres (6) à partir du mélange de polymères et on forme à partir d'elles un semi-produit non-tissé (8), et
   - on produit le non-tissé polymère (1) par production, par extension mécanique du semi-produit non-tissé (8) puis détente, une augmentation de volume.

2. Procédé selon la revendication 1, dans lequel on utilise comme élastomère thermoplastique un polyuréthane thermoplastique.

3. Procédé selon la revendication 1 ou 2, dans lequel on utilise en tant qu'autre matériau thermoplastique un tel matériau qui présente vis-à-vis des composants de l'élastomère thermoplastique une différence de paramètres de solubilité supérieure à 1, de préférence supérieure à $2 \left( \dfrac{1}{cm^3} \right)^{1/2}$.

4. Procédé selon l'une des revendications 1 à 3, dans lequel on utilise comme autre matériau thermoplastique un thermoplastique incompatible avec l'élastomère thermoplastique, par exemple un polypropylène ou un polyéthylène.

5. Procédé selon l'une des revendications 1 à 4, dans lequel l'autre matériau thermoplastique est mélangé à l'élastomère thermoplastique selon une proportion de 1,5 à 30 % en poids, en particulier de 5 à 20 % en poids.

6. Procédé selon l'une des revendications 1 à 5, dans lequel les fibres (6) sont étirées lors de la formation du semi-produit non-tissé (8).

7. Procédé selon l'une des revendications 1 à 6, dans lequel le semi-produit non-tissé (8) est fabriqué par un procédé de fusion-soufflage.

8. Procédé selon l'une des revendications 1 à 7, dans lequel l'extension mécanique provoque une augmentation de la grosseur des pores.

9. Procédé selon l'une des revendications 1 à 8, dans lequel l'épaisseur du semi-produit non-tissé (8) subit, du fait de l'extension mécanique, une augmentation 2 à 10 fois, en particulier 8 fois.

10. Procédé selon l'une des revendications 1 à 9, dans lequel le semi-produit non-tissé (8) subit une extension de 20 à 80 pour cent, en particulier de 25 à 75 pour cent.

11. Utilisation d'un semi-produit non-tissé (8) qui est formé à partir de fibres (6) qui sont produites à partir d'un mélange de polymères qui est formé à partir d'un élastomère thermoplastique et d'un autre matériau thermoplastique, pour fabriquer un non-tissé polymère (1), en particulier une couche de distribution d'acquisition formée à partir de celui-ci, l'extension mécanique du semi-produit non-tissé (8), suivie d'une détente, provoquant une augmentation () du volume du semi-produit non-tissé (8), avec production du non-tissé polymère (1).

12. Utilisation d'un non-tissé polymère (1) qui a été fabriqué par un procédé selon l'une des revendications 1 à 10 comme support de particules de catalyseur, de charbon actif et/ou de superabsorbants.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20190388263 A1
- US 20190183690 A1